(19) Europäisches Patentamt European Patent Office Office européen des brevets

(11) **EP 3 102 181 B1**

(12) **FASCICULE DE BREVET EUROPEEN**

(45) Date de publication et mention de la délivrance du brevet:
**04.07.2018 Bulletin 2018/27**

(21) Numéro de dépôt: **15807961.6**

(22) Date de dépôt: **27.11.2015**

(51) Int Cl.:
*A61K 8/73* (2006.01)          *A61K 8/92* (2006.01)
*A61K 8/97* (2017.01)          *A61Q 19/08* (2006.01)

(86) Numéro de dépôt international:
**PCT/FR2015/053247**

(87) Numéro de publication internationale:
**WO 2016/092179 (16.06.2016 Gazette 2016/24)**

(54) **COMPLEXE ACTIF POUR UN PRODUIT COSMÉTIQUE CONTRE LE VIEILLISSEMENT CUTANÉ**

AKTIVER KOMPLEX FÜR EIN KOSMETIKUM GEGEN HAUTALTERUNG

ACTIVE COMPLEX FOR A SKIN ANTI-AGEING COSMETIC

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.12.2014 FR 1462075**

(43) Date de publication de la demande:
**14.12.2016 Bulletin 2016/50**

(73) Titulaire: **Margnat, Franck**
**13008 Marseille (FR)**

(72) Inventeur: **Margnat, Franck**
**13008 Marseille (FR)**

(74) Mandataire: **Domange, Maxime**
**Cabinet Beau de Lomenie**
**232 Avenue du Prado**
**13008 Marseille (FR)**

(56) Documents cités:
WO-A2-2011/116963          WO-A2-2012/072245
WO-A2-2013/139965          DE-A1-102009 045 981
US-A1- 2009 117 211

• R. JAQUES ET AL: "A SEA ANEMONE EXTRACT (THALASSINE) WHICH LIBERATES HISTAMINE AND A SLOW CONTRACTING SUBSTANCE", BRITISH JOURNAL OF PHARMACOLOGY AND CHEMOTHERAPY, vol. 9, no. 1, 1 mars 1954 (1954-03-01), pages 49-52, XP055202366, ISSN: 0366-0826, DOI: 10.1111/j.1476-5381.1954.tb00815.x
• A BOUKELOUA ET AL: "Acute Toxicity of <em>Opuntia Ficus Indica</em> and <em>Pistacia Lentiscus</em> Seed Oils in Mice", AFRICAN JOURNAL OF TRADITIONAL, COMPLEMENTARY AND ALTERNATIVE MEDICINES, vol. 9, no. 4, 28 septembre 2012 (2012-09-28), XP055202604, DOI: 10.4314/ajtcam.v9i4.19

**EP 3 102 181 B1**

**Description**

La présente invention concerne un complexe actif dénommé anti-âge ayant un effet cutané protecteur, notamment régénérant ou revitalisant, destiné à prévenir ou lutter contre le vieillissement cutané, notamment les rides, le dit complexe comprenant une association d'une pluralité de substances actives.

La présente invention concerne aussi une composition cosmétique ou dermatologique comprenant un dit complexe selon l'invention et des excipients pour une application par voie topique.

La peau comprend une couche superficielle constituée par l'épiderme, et des couches plus profondes formant le derme. La couche superficielle formant l'épiderme est principalement composée de kératinocytes (85 à 90% des cellules épidermiques). Le derme, plus épais, se compose principalement de collagène, d'élastine et de protéoglycanes. Ces trois types de molécules sont synthétisés par les fibroblastes dermiques. Le vieillissement de la peau peut être naturel ou provoqué par l'environnement, y compris les agressions climatiques, qui peuvent notamment contribuer à accélérer la dégradation du collagène du derme, et en particulier l'exposition aux rayons UV du soleil, les variations de températures et les radicaux libres.

On connait de nombreuses compositions ayant des effets de prévention ou lutte contre le vieillissement de la peau, notamment des compositions topiques à base d'extraits végétaux appropriés provenant préférentiellement de plantes connues pour leurs propriétés favorables dans WO 02/060394 et WO 2009/050866.

Cependant, il existe toujours un besoin de pouvoir disposer de nouvelles compositions topiques alternatives permettant de lutter efficacement contre les effets du vieillissement cutané.

L'acide hyaluronique et son sel de sodium sont reconnus pour leurs propriétés hydratantes. La spiruline est une micro-algue très riche en protéines, fer, beta-carotène, vitamines, minéraux, oligo-éléments; elle est connue comme complément alimentaire avec des propriétés antiseptiques. La thalassine est une substance extraite de la plante *Tripleurospermum Maritimum* qui aide à décrisper les muscles du visage et défroisser les traits. L'huile de figue de barbarie est une huile très rare riche en vitamine E et acides gras essentiels qui possède des vertus anti-oxydantes et anti-radicalaires. L'extrait de lys blanc est reconnu pour ses propriétés cicatrisantes et calmantes.

Selon la présente invention, on a cherché à fournir un nouveau complexe de mélange de substances actives ci-après dénommé «mélange d'actif», ayant un effet cutanée antivieillissement, tant au niveau de l'épiderme avec un effet antioxydant, qu'au niveau du derme avec un stimulation de la production de collagène et une diminution des enzymes qui décomposent le collagène, le derme étant considéré comme le siège principal des dommages délétères infligés par le vieillissement.

Le potentiel "anti-âge" des mélanges d'actifs selon la présente invention a été évalué sur des cellules kératinocytes et des fibroblastes humains exposés aux rayonnements ultraviolets par évaluation de leurs effets modulateurs vis-à-vis des altérations fonctionnelles photo-induites de marqueurs biologiques. Le degré d'oxydation épidermique a été évalué par la mesure des espèces réactives oxydantes (connues sous le nom de « ROS ») sur des kératinocytes HaCaT comme marqueur du photo-vieillissement épidermique. La synthèse et dégradation de la matrice extracellulaire ont été évalués par la mesure des taux de procollagène I et MMP-1 sur des fibroblastes NHDF comme marqueurs du photo-vieillissement dermique.

On a déterminé selon la présente invention un indice d'activité anti-âge dénommé « AAC » qui est un paramètre quantitatif du potentiel anti-âge cutanée d'un élément essai prenant en compte un effet protecteur au niveau épidermique en intégrant un score d'effet antioxydant épidermique et un effet protecteur au niveau dermique en intégrant un score d'effet dermo-protecteur vis-à-vis de la synthèse de collagène, marqueur principal de la matrice extracellulaire dermique (MEC) et/ou vis-à-vis de la dégradation de métalloprotéinase-1 (MMP-1) qui est une enzyme collagénase produite par les fibroblastes du derme dégradant la matrice extracellulaire du derme (MEC). Plus précisément encore, on cherche une capacité à s'opposer à la baisse de la synthèse de collagène suite à un stress UV et s'opposer à l'augmentation de la production de metalloprotéinsase-1 pour assurer l'homéostasie dermique.

Plus précisément, selon la présente invention on a défini un potentiel AAC comme suit :

$$AAC == AOE + 1/2 DPC + 1/2\ DPM$$

avec

[AOE] : % potentiel Antioxydant Epidermique défini ci-après.
[DPC.] : % potentiel Dermo-Protecteur Collagène défini ci-après.
[DPM] : % potentiel Dermo-Protecteur MMP1 défini ci-après.

- AOE= % de réduction de ROS après traitement des cellules keratinocytes par UV en présence du complexe selon l'invention par rapport à un témoin ayant subi un même traitement UV en l'absence de complexe d'essai selon la présente invention ou autre.

- DPC=% d'augmentation de collagène après traitement des cellules de fibroblastes NHDF par UV en présence du complexe selon l'invention par rapport à un témoin ayant subi un même traitement UV en l'absence de complexe d'essai selon la présente invention ou autre.

- DPM=% de diminution de métalloprotéinases induits après traitement de fibroblastes NHDF par UV en présence du complexe selon l'invention par rapport à un témoin ayant subi un même traitement UV en l'absence de complexe d'essai selon la présente invention ou autre.

Un autre but de la présente invention était de fournir des complexes anti-âges présentant des indices AAC d'au moins 100, de préférence au moins 110 avec cumulativement :

- AOE au moins égal à 70 de préférence au moins 80, et
- DPC au moins égal à 45, de préférence au moins égal à 50, et
- DPM au moins égal à 15, de préférence au moins égal à 30.

Le document DE 102009045981 décrit l'intérêt d'une composition cosmétique comprenant au moins 13 substances actives (A à M) incluant un extrait de Tripleurospermum et de l'acide hyaluronique. En dépit du très grand nombre de composants additionnels pouvant être inclus dans la composition, il n'est pas fait mention d'extrait d'huile de pépins de figue de barbarie. Dans le document DE 102009045981, aucun résultat sérieux documenté n'est fourni concernant des propriétés anti-ages, que ce soit en termes de potentiel antioxydant épidermique et/ou potentiel thermo-protecteur.

Le document US 2009/116211 décrit des compositions cosmétiques contenant un extrait d'huile de pépins de figue de barbarie. Il est indiqué que cette substance favorise la synthèse de collagène et d'acide hyaluronique au niveau des cellules cutanées. Toutefois, dans le document US 2009/116211, aucune donnée n'est fournie concernant le potentiel antioxydant épidermique AOE ou potentiel dermo-protecteur MMP1 défini ci-dessus de composition testée.

Pour ce faire, la présente invention fournit un complexe actif anti âge pour la peau comprenant au moins les éléments actifs suivants :

- thalassine, extraite de *Tripleurospermum Maritimum*,
- huile de pépins de figue de barbarie, et
- acide hyaluronique ou sel de celui-ci.

Les essais comparatifs fournis dans les exemples détaillés de réalisation ci-après, ont permis d'établir que le potentiel anti-âge des combinaisons selon la présente invention comprenant au moins les 3 éléments actifs définis ci-dessus sont supérieurs à toutes les combinaisons binaires testées.

Plus particulièrement, le complexe selon l'invention sous forme liquide comprend :

- 0,2 à 3% en poids d'une solution aqueuse de thalassine à 2% en poids de thalassine,
- 0,02 à 2% en poids d'huile de figue de barbarie, et
- 0,02 à 0,2% en poids d'acide hyaluronique ou sel de celui-ci.

De préférence, dans les essais comparatifs des exemples ci-après, on a découvert que le potentiel anti-âge cutané le plus élevé était obtenu lorsque le complexe selon l'invention comprend en outre au moins les 2 éléments actifs supplémentaires suivants :

- extrait de fleur de lys blanc *Lilium candidum*, de préférence une solution hydro glycolique de fleur de Lys blanc *Lilium candidum*, et
- spiruline, extraite de *Spirula Maxima,* de préférence une solution hydro glycolique de spiruline.

Plus particulièrement, le complexe selon l'invention sous forme liquide comprend :

- 0,02 à 0,15% en poids de solution hydro glycolique contenant 5% en poids de dit extrait de Lys blanc,
- 0,002 à 0,05% en poids de solution hydro glycolique contenant 1% en poids de spiruline.

Plus particulièrement encore, le complexe selon l'invention sous forme de liquide comprend au moins :

- 1 à 2% en poids de solution aqueuse de thalassine à 2% en poids de thalassine,
- 0,1 à 0,2% en poids de dite huile de figue de barbarie, et.

- 0,1 à 0,2% en poids d'acide hyaluronique ou sel de celui-ci.

Plus particulièrement encore, le complexe selon l'invention sous forme liquide comprend en outre au moins :

- 0,01 à 0,1% en poids solution hydro glycolique contenant 5% en poids de dit extrait de Lys blanc, et
- 0,01 à 0,1% en poids de solution hydro glycolique contenant 1% en poids de spiruline.

Plus particulièrement encore, le complexe selon l'invention sous forme liquide comprend:

- 1,6 % en poids de dite solution de thalassine
- 0,16 % en poids de dite huile de figue de barbarie
- 0,16% en poids d'acide hyaluronique.

Plus particulièrement encore, sous forme liquide comprenant :

- 1,6 % en poids de dite solution de thalassine,
- 0,16 % en poids de dite huile de figue de barbarie,
- 0,16 % en poids d'acide hyaluronique,
- 0,06 % en poids de dite solution hydro glycolique de dit extrait de lys blanc, et
- 0,03 % en poids de dite solution hydro glycolique de spiruline.

La présente invention fournit également un produit cosmétique ou dermatologique comprenant un dit complexe selon l'invention en combinaison avec des supports et/ou excipients cosmétiquement ou pharmaceutiquement acceptables.

On mettra en oeuvre des doses efficaces non cytotoxiques des différents dits éléments actifs du complexe telles que définies ci-dessus.

Plus particulièrement encore, un produit cosmétique antivieillissement selon l'invention est formulé pour une application topique sous forme de gel, lotion, crème, pommade, savon, pâte pour masque.

Les compositions topiques selon l'invention peuvent comprendre divers excipients usuels adaptés à une administration topique externe, en particulier des excipients acceptables sur le plan dermatologique et cosmétologique. Ces excipients appropriés pour la formulation sont bien connus de l'homme du métier et comprennent par exemple des agents favorisant la pénétration, des agents hydratants, des agents épaississants, des agents émollients et des agents tensioactifs, des agents émulsifiants; des agents conservateurs.

Ces formes d'administration par voie topique sont préparées par les techniques connues, et par exemple, dans le cas d'une crème, par dispersion d'une phase grasse dans une phase aqueuse pour obtenir une émulsion huile dans eau, ou inversement pour préparer une émulsion eau dans huile.

Dans un mode particulier de réalisation, le produit cosmétique selon l'invention est formulé sous forme de crème comprenant, les dits éléments du dit complexe et au moins les excipients suivants additionnels: agent émulsifiant, agent émollient, agent épaississant, agent hydratant et agents conservateur et agent ajusteur de Ph.

Dans un mode particulier de réalisation, le produit cosmétique selon l'invention est formulé sous forme de gel aqueux comprenant, en mélange homogène dans de l'eau, les dits éléments du dit complexe et au moins les excipients suivants additionnels : agent gélifiant, agent épaississant, agent hydratant et agent conservateur et agent ajusteur de Ph.

Les préparations contiennent généralement d'autres excipients et/ou auxiliaires et parfois d'autres principes actifs, par exemple des colorants, pigments de coloration, capteurs de radicaux, parfums, agents stabilisants.

Les produits cosmétiques peuvent aussi comprendre des auxiliaires et parfois d'autres principes actifs, par exemple vitamines antioxydantes telles que la vitamine E, la vitamine C, des agents antioxydants comme les polyphénols naturels, des enzymes, principes actifs végétaux, substances anti-inflammatoires naturelles, des alcools, polyols, esters, électrolytes, huiles polaires et non polaires, des polymères, copolymères, phopholipides, des colorants; des parfums; ou encore des agents de gommage de la peau.

L'invention a encore pour objet un procédé cosmétique de traitement de la peau pour lutter contre les signes du vieillissement cutané, et en particulier les rides d'expression provoquées par les contractions musculaires faciales incontrôlées, consistant à appliquer sur les zones de la peau nécessitant un tel traitement, un produit cosmétique topique selon l'invention.

D'autres caractéristiques et avantages de la présente invention ressortiront mieux à la lecture de la description des exemples qui vont suivre, faite de manière illustrative et non limitative.

EXEMPLE 1 : COMPLEXES ACTIF ANTI-AGES

On a évalué ci-après la cytotoxicité et le potentiel anti-âge de plusieurs complexes (mélanges) d'actifs sur des cellules cutanées humaines en culture.

1) On a testé différents mélanges complexes à partir des éléments actifs suivants.

1.1) Solution aqueuse de thalassine.

La thalassine est extrait de la plante *Tripleurospermum Maritimum* de la famille des Asteraceae composée des éléments suivants aux teneurs suivantes :

- Acides aminés : Alanine (10.32%), Glycine (1.64%), Valine (7.14%), Leucine (4.82%), IsoLeucine (3.45%), Thréonine (2.22%), Proline (23.18%), Asparagine (10.56%), acide aspartique (12.63+%), Phénlyalanine (4.64%), Tyrosine (0.92%),
- Fructo-oligosaccharides : fructanes à raison de 0.08% en poids,
- Vitamines B5 ($0.03 \times 10^{-3}$%), B6 ($33.5 10^{-6}$ %), PP ($0.27 \, 10^{-3}$%), B9 ($3.10^{-6}$%),
- Sucres : fructose ($90.5 \times 10^{-3}$ %), saccharose ($32.4 \times 10^{-3}$%),
- Minéraux : Ca ($9.9 \times 10^{-3}$%), Mg ($4.8 \times 10^{-3}$%), Na ($9.8 \times 10^{-3}$%), K ($134.9 \times 10^{-3}$%), Zn ($0.05 \times 10^{-3}$%) et Mn ($0.02 \times 10^{-3}$%).

Il s'agit d'un extrait liquide aqueux commercialisé par la société BIOTECH MARINE (France Z.I. BP 72 2260 PONTRIEUX) sous la référence THALASSINE 2. Un tel actif contient les éléments listés ci-dessus aux teneurs listés de l'extrait de *Tripleurospermum Maritimum* ci-dessus dans une teneur de 2% en poids en solution dans de l'eau (97.8%) et de l'acide sorbique (0.2% en poids).

1.2) Solution hydro glycolique de fleur de Lys blanc ou *Lilium candidum.*

Le liquide utilisé est commercialisé par la société IES LABO France 04700 ORAISON) sous la référence EH354.Cette solution est obtenue par filtration après macération longue et agitation du végétal dans un mélange d'eau et propylène glycol à température ambiante. Après retrait de la plante, une telle solution contient % en poids :

- 5% d'extrait de de la plante,
- 45% de propylène glycol,
- 45% d'eau.

Les constituants extraits de *Lilium candidus* sont des actifs lipophiles comprenant une huile essentielle riche en composés aromatiques, des phytostérols (spirostane, furostane, béta-sitostérol) et des alcaloïdes stéroïdiens (étioline).
Ses caractéristiques physico-chimiques sont :

- aspect: liquide marron à jaune,
- densité à 20°C : 1050 à 1.070 g/cm$^3$
- PH=3.5 à 5.5 ;
- indice de réfraction à 20°C :1.68 à 1.410.

1.3) Solution hydro glycolique de spiruline.

La spiruline est extraite d'une micro-algue que l'on trouve dans les lacs de la ceinture intertropicale. Elle est constituée essentiellement à environ 50-75% de protéines végétales, en mélange avec des vitamines A, E, B1, B2, B3, B6, B7, B8, K et de béta-carotène, de minéraux et oligo-éléments tels que Ca, P, Mg , Fe, Zn, Cu, Mn, Cr, Na, K, et Se. Elle est très riche en chlorophylle et phycocyanine. Elle comprend aussi des enzymes dont la superoxyde dismutase (SOD) qui contient du Fe. Enfin, elle contient des acides gras essentiels : oméga 6, acide gamma-linolénique en grande quantité.
La solution liquide utilisée est une solution hydroglycolique de spiruline contenant un extrait de *Spirulina* maxima commercialisé par la société GREENTECH.FRANCE 63360 SAINT-BEAUZIRE) sous la référence 300656. Une telle solution contient en % en poids:

- 1% d'extrait sec de *Spirulina Maxima*

- 49.5 % d'eau, et
- 49.5% de butylène glycol.

1.4) huile de pépins de figue de barbarie.

Le figuier de barbarie est une sorte de cactus (*Opuntia ficus-indica*) qui produit des pépins dont on extrait une huile riche en vitamine F et acide alpha-linolénique, vitamine E et stérols.

L'huile utilisée est commercialisée par la société IES LABO France 04700 ORAISON sous la référence HV141. Cette huile est obtenue par pression à froid des graines de la plante originaire de Tunisie. L'huile contient :

- 10 à 14 % d'acide palmitique en C16:0;
- 1% d'acide palmitoléique en C 16:1;
- 2.5 à 5.5% d'acide stéarique C18:0;
- 20 à 30% d'acide oléique en C18:1;
- 55 à 65% d'acide linoléique en C18:2,
- 1% d'acide linolénique en C18:3.

1.5) Acide hyaluronique.

L'acide hyaluronique est un polymère glycosaminoglycane de $10^2$ à $10^4$ KDa constitué d'unités disaccharidiques de type acide D-glucuronuique et D-N-acétylglucosamine, les liaisons glycosidiques étant alternées: béta 1-4 et béta 1-3.

L'acide hyaluronique a été utilisé sous forme poudre contenant plus de 91% en poids d'hyaluronate de sodium commercialisée par exemple par la société SHANDONG FOCUSCHEM BIOTECH CO. LTD, CHINA). Il est produit par fermentation bactérienne avec la bactérie *Streptococcus zooepidemicus.*

2) CYTOTOXICITE.

Pour chacune des substances ci-dessus on a déterminé les doses maximales non cytotoxiques correspondant à viabilité cellulaire $\geq$ 90% (ci-après « DNC »). La viabilité cellulaire a été évalué par un test Rouge Neutre (RN) sur des cellules kératinocytes humains (lignée HaCaT) et fibroblastes décrites ci-après après contact de 48h. Les substances étaient versées dans le milieu de culture DMEM + SVF 10%.

| RESULTATS | |
|---|---|
| Eléments d'essai | DNC (%) En poids |
| Solution de THALASSINE | 3% |
| Solution HYDRO GLYCOLIQUE DE LYS DE FLEUR | 0.15% |
| Solution HYDRO GLYCOLIQUE DE SPIRULINE | 0.05% |
| HUILE DE FIGUE DE BARBARIE | 2% |
| HYALURONATE DE SODIUM POUDRE | 0.2% |

3) ACTIVITE ANTI-AGE.

On a évalué ci-après le potentiel anti-âge de plusieurs complexes (mélanges) d'actifs sur des cellules cutanées humaines en culture. Une approche expérimentale in vitro basée sur la production des ROS par des kératinocytes épidermiques humains après exposition aux UV-B a été proposée pour apprécier le potentiel anti-oxydant des complexes de mélanges d'actifs. Les effets dermo-protecteurs des complexes ont été appréciés par la mesure des taux de collagène I et MMP-1 sur des fibroblastes dermiques humains soumis aux UV.

Les complexes de mélanges [CAAL-2], [CAAL-3] et [CAAL-4] ont été préparés directement dans le milieu de culture, à partir des différents actifs susnommés, selon les formules du tableau I ci-après avec les concentrations en % en poids.

Tableau I

| COMPLEXE ANTI-AGE | | CAAL-2 | CAAL-3 | CAAL-4 |
|---|---|---|---|---|
| THALASSINE | | 1.6% | 1.6% | - |
| SOLUTION HYDRO GLYCOLIQUE DE LYS | | 0.06% | - | 0.06% |
| SOLUTION HYDRO GLYCOLIQUE DE SPIRULINE | | 0.03% | - | 0.03% |
| HUILE DE FIGUE DE BARBARIE | | 0.16% | 0.16% | 0.16% |
| ACIDE HYALURONIQUE | | 0.16% | 0.16% | - |

L'étude a été réalisée sur des fibroblastes dermiques humains [NHDF] et des kératinocytes humains HaCaT.

Les cellules NHDF sont des fibroblastes de derme humain en primoculture qui ont été isolés et qui sont conservés et cultivés au laboratoire. Les cellules [NHDF] isolées à partir de peau de prépuce de jeunes enfants sont cultivées en milieu D-MEM (Gibco®) supplémenté en sérum de veau foetal (SVF 10%) et en antibiotiques, selon les techniques mises en place au laboratoire, à 37°C en atmosphère humide air-CO2 (95%-5%).

Les cellules HaCaT sont des kératinocytes humains issus d'une lignée cellulaire immortalisée qui est conservée au laboratoire (Normal Keratinization in a Spontaneously Immortalized Aneuploid Human Keratinocyte Cell Line N.E Fusenig, J. Cell. Biology; 106, 1988). Les cellules HaCaT sont cultivées, en milieu D-MEM (Gibco®) supplémenté en sérum de veau foetal (SVF 10%) et en antibiotiques, à 37°C en atmosphère air-CO2 (95%-5%).

Les cellules HaCaT et NHDF sont cultivées dans des flacons de 25 ou 75 cm2 et sont passées régulièrement avant d'atteindre la confluence.

3.1) POTENTIEL ANTI-OXYDANT EPIDERMIQUE (ci-après «AOE»).

3.1.1) Le principe du test repose sur la mesure du degré d'oxydation intracellulaire à l'aide de la sonde : 2',7'-dichloro-dihydrofluorescein diacétate (DCFH-DA). DCFH-DA non fluorescent, pénètre par diffusion passive dans les cellules. Après clivage des groupements acétate par les estérases, DCFH s'accumule au niveau du cytosol. L'oxydation de DCFH par les espèces réactives de l'oxygène (ROS:« Reactive oxydative species ») conduit à la formation d'un composé fluorescent. L'intensité de fluorescence de DCF permet d'évaluer le degré d'oxydation des cellules soumises à un stress oxydatif.

Plus précisément, la mesure des intensités de fluorescence de DCF permet d'évaluer le degré d'oxydation des cellules soumises à un stress oxydatif. Le test DCFH détecte une large gamme de ROS: hydroxyl peroxyl et autres ROS (Fluorescence probes used for détection of reactive oxygen species, A. Gomes et al., J. Biochem. Biophys. Methods, 65:45-80; 2005).-

Des kératinocytes HaCaT cultivés en monocouche sont détachés par trypsinisation. La suspension cellulaire est ensemencée dans des plaques 24 puits.

2 plaques sont ensemencées en parallèle : 1 plaque [DCFH] (mesure de la fluorescence DCFH) et 1 plaque [Prot] (mesure des protéines cellulaires).

Les plaques [DCFH] et [Prot] sont traitées en parallèle, de manière identique, selon le protocole expérimental suivant.

72h après ensemencement, le milieu de culture est éliminé et remplacé par du milieu neuf contenant l'élément d'essai. Les cellules sont replacées à 37°C et incubées pendant 24h, en atmosphère air-CO2 (95%/5%). Après incubation avec l'élément d'essai, les cellules sont rincées avec du HBSS (solution dite de Hanks) puis exposées aux UV-B (tubes Philips TL-K 40W ACTINIC BL REFLECTOR) à travers du HBSS. Les cellules ont été traitées avec les différents complexes testés avant (24h) et après (24h) irradiation UV-B d'environ 30 minutes à 20mJ/cm2.

Après irradiation, le HBSS est remplacé par du milieu neuf contenant l'élément d'essai. Les cellules sont replacées à 37°C pendant 24h en atmosphère air-CO2 (95%/5%). En fin d'essai, les plaques [DCFH] et [Prot] sont traitées, de la manière suivante :

▪ Plaque [DCFH]:

Les plaques [DCFH] sont vidées et rincées avec du PBS-. Les cellules sont ensuite mises à incuber à 37°C, en présence d'une solution de DCFH-DA. Après incorporation de la sonde, les cellules sont rincées avec du PBS-. Un tampon d'extraction (Triton X-100 1%) est ajouté dans chaque puits. Après 45mn d'incubation, les lysats sont prélevés, transférés dans des plaques 96-puits. Les intensités de fluorescence (·Exc : 485nm, ·Em : 535nm) sont exprimées en unité relative de fluorescence (URF).

▪ Plaque [Prot] :

Un dosage des protéines (BCA Protein assay) est réalisé en parallèle afin de corriger les valeurs [DCFH] (URF/puits) en fonction du taux de protéines cellulaires (μg/puits) pour s'affranchir d'une variation du nombre de cellules liée à un effet de l'élément d'essai sur la croissance cellulaire.

3.1.2) Résultats

L'essai a été réalisé sur des kératinocytes HaCaT (souche HaCaTp91) cultivés en milieu [DMEM +1% SVF]. Le degré d'oxydation intracellulaire a été mesuré après traitement et irradiation des cellules.
Les 3 complexes de mélanges dénommés CAAL-2, CAAL-3 et CAAL-4 ont été testés comprenant les concentrations en différents actifs indiquée dans le tableau I ci-dessus.
La Vitamine E (Tocopherol, 1mM) a été testée en parallèle comme contrôle positif.
Le degré d'oxydation intracellulaire (U.R.F/puits, U.R.F (R.F.U) : unité relative de fluorescence (relative fluorescence unit) enregistré au niveau des cultures témoins et traitées a été corrigé en fonction du taux moyen des protéines cellulaires (μg/puits). Les valeurs [DCFH] sont exprimées en unité arbitraire (à partir des densités optiques):[DCFH] = (U.R.F/μg prot).
Les données sont consignées dans le tableau II.
Les résultats (moyenne +/- écart-type, n=3) sont collectés dans le tableau ci-après.
Les valeurs « stat. » p obtenues par le test de student t sont inférieures ou égales à 0,01.

| Tableau II | | DCFH ASSAY | ROS induits par UV | | Stat. |
|---|---|---|---|---|---|
| (-) UV | Témoin 1 | 12.80 +/- 0.99 | | (%) | |
| (+) UV | Témoin 2 | 134.12 +/- 2.23 | 121.32 | 100% | |
| | [CAAL-2] | 18.12 +/- 0.96 | 5.32 | 4% | p≤0.01 |
| | [CAAL-3] | 45.31 +/- 4.81 | 32.51 | 27% | p≤0.01 |
| | [CAAL-4] | 74.31 +/- 4.23 | 61.51 | 61% | p≤0.01 |
| | VE | 35.72 +/- 2.66 | 22.92 | 19% | p≤0.01 |

L'exposition des cellules témoins aux UVB se traduit par une très nette augmentation du degré d'oxydation intracellulaire. Le taux basal des ROS (cultures non irradiées : témoin 1) est multiplié par 10.5 après exposition UV des cellules (témoin 2).
Les résultats montrent que le traitement des cellules par [CAAL-2], [CAAL-3] et [CAAL-4] permet de réduire très nettement les ROS induits par les UV ([ROSUV]) par rapport à un témoin (témoin 2) ayant subi le même traitement UV mais en l'absence de complexe d'essai selon la présente invention ou autre. Il convient de noter que le potentiel antioxydant épidermique varie selon le mélange considéré comme suit:

- CAAL-2 : AOE= 96% de réduction de [ROSUV]
- CAAL-3 : AOE=73% de réduction de [ROSUV]
- CAAL-4 : AOE=39% de réduction de [ROSUV]

AOE= % de réduction de ROS induits après traitement des cellules keratinocytes par UV en présence du complexe selon l'invention par rapport à un témoin ayant subi un même traitement UV en l'absence de complexe d'essai selon la présente invention ou autre.
Les différences enregistrées au niveau des lots CAAL-2, CAAL-3 et CAAL-4 sont statistiquement significatives par rapport au témoin (+) UV (p≤0.01, test t de Student). L'effet antioxydant maximal est enregistré avec [CAAL-2] qui s'oppose presque complétement au stress oxydatif induit par les UV.
Dans les mêmes conditions expérimentales, le traitement des cellules HaCaT par la Vitamine E (-tocopherol, 1mM) permet de réduire de 81% le stress oxydatif induit par les UV.

3.2) POTENTIEL DERMO-PROTECTEUR.

3.2.1) Le principe du test repose sur l'évaluation de 2 paramètres - néosynthèse de collagène I et production de MMP-1 - sur des fibroblastes humains [NHDF] cultivés en absence ou présence de l'élément d'essai, et stimulés par les UV-A.

Le taux de pro-collagène I est quantifié par dosage de [PIP] (Procollagen type I Peptide) dans les milieux d'incubation à l'aide d'un test ELISA. La mesure du taux de MMP-1 est effectuée sur les milieux d'incubation à l'aide d'un test Elisa.

Des fibroblastes [NHDF] sont détachés de leur support par trypsinisation. Après numération, la suspension cellulaire est ensemencée dans des plaques 24-puits à raison de $1.10^5$ cell./puits, en milieu [D-MEM + 10% SVF].

Après 24h d'incubation, le milieu de culture est éliminé et remplacé par du milieu neuf supplémenté avec 1% de SVF, dépourvu d'acide ascorbique et contenant le(s) produit(s) à l'étude. Les cellules sont replacées à l'étuve et incubées, pendant 24h, en atmosphère air-CO2 (95%/5%).

Après incubation avec les solutions-tests, les cellules sont rincées avec du HBSS puis exposées aux UV-A (tubes Philips TL-K 40W ACTINIC BL REFLECTOR) à travers du HBSS. L'irradiation en UV-A est d'environ 10J/cm21 pendant 1h. Après irradiation, le HBSS est remplacé par du milieu neuf contenant l'élément d'essai.

Les cellules sont replacées à 37°C et incubées pendant 24h. A la fin de l'essai, les milieux d'incubation des cultures sont prélevés et congelés à -20°C en attente du dosage du procollagène I et de MMP-1.

Parallèlement, les tapis cellulaires sont rincés avec du HBSS et traités pour un dosage des protéines cellulaires (BCA Protein assay). Une solution de NaOH (0.1N) est ajoutée dans chaque puits. Après incubation, les extraits sont prélevés et transférés dans une plaque 96-puits. 200$\mu$l de réactif BCA sont ajoutés dans chaque puits. Après incubation, l'absorbance (densité optique) est mesurée à 570nm avec un lecteur de microplaques.

Le dosage du procollagène I dans les échantillons est réalisé avec un test ELISA (Procollagen Type I C-Peptide EIA Kit de laboratoires R&D Systems®, France) de détection et quantification par fluorescence du peptide C-terminal du procollagène I humain.

L'activité MMP-1 est mesurée avec un test ELISA (Human Active MMP-1 de laboratoires R&D Systems®, FRANCE) de détection et quantification par fluorescence de l'activité MMP-1.

Dans les deux cas la quantification de la fluorescence se fait dans les surnageants de culture, selon les instructions fournies par les laboratoires R&D Systems®, France, l'absorbance étant mesurée à 450nm.

3.2.2) Résultats.

L'essai a été réalisé sur des fibroblastes [NHDF] (souche F06.2H7) cultivés en milieu [DMEM + 1%SVF], en absence d'ascorbate de sodium.

Les taux de procollagène I et de MMP-1 ont été mesurés dans les surnageants des cultures après traitement et irradiation des cellules.

Les cellules NHDF ont été traitées avec les complexes d'essai CAAL-2, CAAL-3 et CAAL-4, avant (24h) et après (24h) irradiation UVA. Les mélanges ont été testés à la concentration indiquée au tableau I ci-dessus.

La Vitamine E (-tocopherol, 1mM, ci-après « VE ») et l'ascorbate de Na (50$\mu$g/ml) ci-après «ASC» ont été testés en parallèle comme contrôles positifs.

Les concentrations en peptide c-terminal de procollagène I [PIP] et de métalloprotéinase-1 [MMP-1] dans les différents échantillons (ng/ml) ont été corrigées en fonction des concentrations protéiques des puits correspondants.

Les résultats (moyenne +/- écart-type, n=3) sont collectés dans le tableau III ci-après.

Le potentiel dermo-protecteur est déterminé ici par ½ DPC + ½ DPM avec :

- DPC=% d'augmentation de collagène après traitement des cellules de fibroblastes NHDF par UV en présence du complexe selon l'invention par rapport à un témoin ayant subi un même traitement UV en l'absence de complexe d'essai selon la présente invention ou autre; et
- DPM=% de diminution de métalloprotéinase-1 après traitement de fibroblastes NHDF par UV en présence du complexe selon l'invention par rapport à un témoin ayant subi un même traitement UV en l'absence de complexe d'essai selon la présente invention ou autre.

Tableau III

|  |  | [PIP] | (%) | Stat | [MMP-1] | (%) | Stat |
|---|---|---|---|---|---|---|---|
| (-) UV | Témoin 1 | 2.21+/0.011 | 160% |  | 0.052+/0.001 | 21% |  |

9

(suite)

|  |  | [PIP] | (%) | Stat | [MMP-1] | (%) | Stat |
|---|---|---|---|---|---|---|---|
| (+) UV | Témoin 2 | 1.38+/0.012 | 100% |  | 0.248+/0.002 | 100% |  |
|  | [CAAL-2] | 2.13+/0.011 | 154% | p≤0.01 | 0.201+/0.002 | 81% | p≤0.01 |
|  | [CAAL-3] | 2.02+/0.002 | 146% | p≤0.01 | 0.167+/0.001 | 67% | p≤0.01 |
|  | [CAAL-4] | 1.70+/0.033 | 123% | p≤0.01 | 0.266+/0.003 | 107% | p≤0.01 |
|  | [ASC] 50μg/ml | 3.36+/0.016 | 243% | p≤0.01 | -t |  |  |
|  | [VE] 1mM | - |  |  | 0.172+/- | 69% | p≤0.01 |

a) Procollagène I.

L'irradiation UVA (20J/cm2) induit une nette baisse (-38%=(160-100/160)) de la synthèse de procollagène

Dans les conditions expérimentales retenues, les complexes d'essai [CAAL-2], [CAAL-3] et [CAAL-4] induisent tous une augmentation significative de [PIP], variable selon le complexe considéré par rapport à un témoin non ayant subi le même traitement UV mais en l'absence de complexe d'essai selon la présente invention ou autre. La plus forte augmentation est enregistrée avec [CAAL-2] qui est capable de restaurer totalement la synthèse du procollagène I déprimée par les UV.

Les 3 complexes testés peuvent être classés en fonction du potentiel dermo-protecteur vis-à-vis du collagène (DPC) de la manière suivante :

$$\text{CAAL-2] (DPC=+54\%)} > \text{[CAAL-3] (DPC=+46\%)} \gg \text{[CAAL-4] (DPC=+23\%)}$$

Dans les mêmes conditions expérimentales, l'ascorbate de sodium ([ASC], 50μg/ml) est capable de restaurer la néosynthèse du collagène I déprimée par les UV avec un score DPC=143%.

b) MMP-1.

L'exposition UVA des cellules NHDF se traduit par une très forte augmentation de MMP-1.

Les éléments d'essai [CAAL-2] et [CAAL-3] induisent une diminution significative de MMP-1. L'effet maximal est enregistré après traitement par [CAAL-3] avec DPM=33% de réduction de MMP-1.

A l'inverse, le mélange [CAAL-4] ne manifeste pas d'effet dermo-protecteur significatif

Les 3 complexes testés peuvent être classés en fonction du potentiel dermo-protecteur vis-à-vis de MMP-1, de la manière suivante :

$$\text{CAAL-3] (DPM=+34\%)} > \text{[CAAL-2] (DPM=+19\%)} \gg \text{[CAAL-4] (DPM≈0\%)}$$

Dans les mêmes conditions expérimentales, la Vitamine E (VE, 1mM) réduit le taux de MMP-1 induit par les UV avec un score de DPM=31%.

4) POTENTIEL ANTI-AGE CUTANEE (ci-après « AAC »).

Le potentiel anti-âge cutané ([AAC]) a été calculé à partir des données expérimentales selon :

$$[A.A.C] = [AOE] + \tfrac{1}{2}.[DPC] + \tfrac{1}{2}.[DPM]$$

avec :

[AOE] : % potentiel Antioxydant Epidermique défini ci-dessus.
[DPC.] : % potentiel Dermo-Protecteur Collagène défini ci-dessus.
[DPM.] : % potentiel Dermo-Protecteur MMP1 défini ci-dessus.

Les résultats des 3 mélanges testés sont collectés ci-dessous.

- CAAL-2 : AOE=96%, DPC=54%, DPM=19, AAC=132.5%;
- CAAL-3 : AOE=73%, DPC=46%, DPM=34%, AAC=113%;
- CAAL-4 : AOE=39, DPC=23, DPM=0, AAC=50.5 ;

Les contrôles positifs VE et ASC sont des molécules de référence pour chaque compartiment: ASC pour le compartiment épidermique et VE pour le compartiment dermique. Néanmoins, si on calcule leurs indices [A.A.C], on obtient pour VE: [AAC] = 96.5 et pour ASC [AAC] = 71.5. (VE : AOE=81%, DPC=0, DPM=31%, AAC= 96.5% et ASC : AOE=0, DPC= 143%, DPM= 0, AAC=71.5%).

Dans les conditions expérimentales retenues, les résultats de l'étude indiquent que les 3 complexes manifestent un potentiel anti-âge significatif qui varie nettement selon le complexe considéré. Les résultats permettent de classer, en fonction du potentiel anti-âge, les 3 complexes de la manière suivante :

$$\text{CAAL-2] > [CAAL-3] >> [CAAL-4}$$

Sur la base des biomarqueurs retenus (ROS, pro-collagène et MMP-1), et compte tenu de leur rôle important dans le processus du vieillissement cutané, la capacité manifestée par le complexe [CAAL-2], d'une part, à moduler la production des ROS au niveau épidermique et, d'autre part, à stimuler la néo-synthèse du collagène et réduire la production des MMPs au niveau dermique apparaît comme très favorable et permet de conclure à l'activité anti-âge recherchée la plus élevée.

Le mélange [CAAL-2] avec un [AAC] de 132.5 présente le score le plus élevé des 3 mélanges testés. L'intérêt des complexes CAAL-2 et CALL-3 est l'efficacité anti-âge au niveau des 2 compartiments cutané épidermique et dermique d'une part et en ce qui concerne le derme à la fois une augmentation du collagène et une diminution de metalloprotéinase-1.

EXEMPLE 2 : ETUDE COMPARATIVE DU POTENTIEL ANTI-AGE DU COMPLEXE [CAAL-3] PAR RAPPORT A 3 COMBINAISONS BINAIRES D'ACTIFS

On a testé les 4 combinaisons de mélange suivantes avec les ingrédients et selon le protocole décrits dans l'exemple 1.

M1 : complexe [CAAL-3]
M2 : combinaison binaire [thalassine + figue barbarie]
M3 : combinaison binaire [thalassine + acide hyaluronique]
M4 : combinaison binaire [figue barbarie + acide hyaluronique]

Les 4 mélanges expérimentaux [M1], [M2], [M3] et [M4] ont été préparés directement dans les milieux de culture, à partir de 3 actifs selon les formules ci-après :

| Mélanges expérimentaux | M1 | M2 | M3 | M4 |
|---|---|---|---|---|
| Actifs | 15.736 | 15.737 | 15.738 | 15.739 |
| THALASSINE 2 | 1.6% | 1.6% | 1.6% | - |
| HUILE VEGETALE DE FIGUE DE BARBARIE | 0.16% | 0.16% | - | 0.16% |
| ACIDE HYALURONIQUE | 0.16% | - | 0.16% | 0.16% |

I. POTENTIEL ANTI-OXYDANT EPIDERMIQUE - DCFH assay :

Etude réalisée sur Kératinocytes humains HaCaT

Conditions d'essai :

1. Prétraitement des cellules, pendant 24 h, avec les éléments d'essai, avant irradiation des cellules,
2. Exposition des cellules aux UV-B (20mJ/cm2), en absence des éléments d'essai,
3. Incubation des cellules irradiées pendant 24h, en présence des éléments d'essai,
4. Quantification du degré d'oxydation intracellulaire (ROS) par mesure de la fluorescence de DCFH.

Résultats :

Le degré d'oxydation intracellulaire (U.R.F/puits) enregistré au niveau des cultures Témoins et Traitées a été corrigé en fonction du taux moyen des protéines cellulaires ($\mu$g/puits). Les valeurs finales ([ROS]) sont exprimées en unité arbitraire :

$$[ROS] = (U.R.F/\mu g\ prot)\ avec\ U.R.F = Unité\ Relative\ de\ Fluorescence$$

Lots Traités [MELANGES] : les 4 éléments d'essai ont été testés à 100% dans milieu [DMEM + SVF(1%)]
Les résultats (moyenne +/- écart-type, n=3) sont collectés dans le tableau ci-après.

| Tableau IV | | DCFH | | | Stat |
|---|---|---|---|---|---|
| (-) UV | | 11.65 +/- 0.70 | | (%) | |
| (+) UV | | 152.17 +/- 5.67 | 140.52 | 100% | |
| | | 52.89 +/- 1.77 | 41.24 | 29% | p$\leq$0.01 |
| | | 68.51 +/- 1.09 | 56.86 | 40% | p$\leq$0.01 |
| | | 89.88 +/- 2.05 | 78.23 | 56% | p$\leq$0.01 |
| | | 106.51 +/- 1.13 | 94.86 | 62% | p$\leq$0.01 |
| | | 28.03 +/- 1.12 | 16.38 | 12% | p$\leq$0.01 |

L'exposition des cellules HaCaT aux UV se traduit par une très nette augmentation du degré d'oxydation intracellulaire. Le degré d'oxydation basal (cultures non irradiées) est multiplié par 13 après exposition UV des cellules.
Les résultats montrent que le traitement des cellules par les différentes combinaisons [M1], [M2], [M3] et [M4] permet de réduire très nettement le taux de ROS intracellulaires induits par les UV (ROSUV). Il convient de noter que le potentiel antioxydant épidermique (AOE) varie nettement selon le mélange considéré et que celui-ci est le plus élevé pour le mélange ternaire :

- M1 : AOE = 71% de réduction de [ROSUV] (p$\leq$0.01, test t de Student)
- M2: AOE = 60% de réduction de [ROSUV] (p$\leq$0.01, test t de Student)
- M3: AOE = 44% de réduction de [ROSUV] (p$\leq$0.01, test t de Student)
- M4: AOE = 38% de réduction de [ROSUV] (p$\leq$0.01, test t de Student)

Dans les mêmes conditions expérimentales, le traitement des cellules par la Vitamine E ($\alpha$-Tocopherol, 1mM) permet de réduire de 88% le stress oxydatif induit par les UV. Ce résultat valide l'essai.

II. POTENTIEL DERMOPROTECTEUR :

Etude réalisée sur fibroblastes dermiques humains (NHDF)

Conditions d'essai :

1. Traitement des cellules, pendant 24 h, avec les éléments d'essai, avant irradiation des cellules,
2. Exposition des cellules aux UV-A, en absence des éléments d'essai,
3. Incubation des cellules irradiées pendant 24h, en présence des éléments d'essai,
4. Quantification des taux de pro-collagène I (dosage [PIP]) et de MMP-1 dans les milieux d'incubation.

Résultats : Les valeurs de [PIP] et de [MMP] enregistrées au niveau des cultures Témoins et Traitées ont été corrigées en fonction du taux moyen des protéines cellulaires ($\mu$g/puits).

Synthèse de procollagène I: dosage du taux de [PIP] (Procollagen type I Peptide) dans les milieux d'incubation. contrôle positif: sodium ascorbate (ASC, 50$\mu$g/ml)

Production de MMP-1: dosage de MMP-1 dans les milieux d'incubation par test Elisa. contrôle positif: vitamine E (VE, 1mM)

Lots traités : les 4 éléments d'essai ont été testés à 100% dans milieu [DMEM + SVF(1%)].

| Tableau V | | [PIP] | | Stat | [MMP-1] | | Stat |
|---|---|---|---|---|---|---|---|
| (-) UV | | 1.99 +/- 0.02 | 128% | | 0.036 +/- 0.002 | 11% | |
| (+) UV | | 1.56 +/- 0.04 | 100% | | 0.317 +/- 0.008 | 100% | |
| | | 1.85 +/- 0.03 | 119% | p$\leq$0.01 | 0.189 +/- 0.010 | 60% | p$\leq$0.01 |
| | | 1.58 +/- 0.02 | 101% | n.s | 0.184 +/- 0.004 | 58% | p$\leq$0.01 |
| | | 1.54 +/- 0.01 | 99% | n.s | 0.210 +/- 0.003 | 66% | p$\leq$0.01 |
| | | 1.65 +/- 0.04 | 106% | n.s | 0.175 +/- 0.004 | 55% | p$\leq$0.01 |
| | | 3.79 +/- 0.04 | 243% | p$\leq$0.01 | n.t | | |
| | | n.t | | | 0.198 +/- 0.006 | 62% | p$\leq$0.01 |

a) Synthèse Collagène I : l'irradiation UVA induit une baisse de la synthèse du collagène. Dans les conditions expérimentales retenues, seul l'élément d'essai [M1] induit une augmentation de [PIP] significative par rapport au contrôle (+)UV. L'élément d'essai [M1] est capable de restaurer en partie la néosynthèse du collagène I déprimée par les UV.

Les 4 complexes testés peuvent être classés, en fonction du potentiel dermo-protecteur vis-à-vis du collagène (DPC), de la manière suivante:

$$[M1] \ (DPC = +19\%) >> [M2] \approx [M3] \approx [M4] = (DPC \approx 0\%)$$

b) Production de MMP-1: l'exposition UVA des cellules NHDF se traduit par une très forte augmentation de la production de MMP-1. Les éléments d'essai [M1], [M2], [M3] et [M4] induisent une diminution significative de MMP-1. Il convient de noter que l'effet dermo-protecteur (MMP-1) est quasiment similaire avec les 4 mélanges. L'effet max est enregistré avec [M4] : 45% de réduction de MMP-1. Les 4 éléments d'essai peuvent être classés, en fonction du potentiel dermo-protecteur vis-à-vis de MMP-1 (DPM), de la manière suivante:

$$[M4] \ (DPM = 45\%) \geq [M2] \ (DPM = 42\%) \approx [M1] \ (DPM = 40\%) > [M3] \ (DPM = 34\%)$$

■ Lot Traité [ASC]: Dans les mêmes conditions expérimentales, l'ascorbate de sodium (50$\mu$g/ml) est capable de restaurer la néosynthèse du collagène I déprimée par les UV.

■ Lot Traité [VE]: Dans les mêmes conditions expérimentales, la Vitamine E (1mM) réduit de 38% (test de Student, p $\leq$0.01) le taux de MMP-1 induit par les UV.

CONCLUSION : Le potentiel Anti-âge cutané des 4 mélanges expérimentaux a été calculé à partir des données expérimentales selon :

$$[A.A.C] = [AOE] + \frac{1}{2}.[DPC] + \frac{1}{2}.[DPM]$$

| Tableau VI | M1 | M2 | M3 | M4 |
|---|---|---|---|---|
| (AOE) potentiel antioxydant épidermique | 71 | 60 | 44 | 38 |

(suite)

| Tableau VI | M1 | M2 | M3 | M4 |
|---|---|---|---|---|
| (DPC) potentiel dermo-protecteur collagène | 19 | 0 | 0 | 0 |
| (DPM) potentiel dermo-protecteur MMP1 | 45 | 42 | 40 | 34 |
| (AAC) potentiel anti-âge cutané | 103 | 81 | 64 | 55 |

Le potentiel anti-âge de la combinaison ternaire M1 est donc supérieur à celui des 3 combinaisons binaires M2, M3 et M4.

EXEMPLE 3 : COMPOSITIONS COSMETIQUES POUR APPLICATION TOPIQUE

1) CREME

On a préparé une crème à partir du mélange d'actifs CAAL-2 de composition suivante :

| NOM COMMERCIAL | NOM INCI | FOURNISSEUR | % en poids |
|---|---|---|---|
| | EAU PURIFIEE | | 78,69000 |
| | DISODIUM EDTA | | 0,10000 |
| | GLYCERINE | | 5,00000 |
| SENSIVA ®SC 50 | ETHYLHEXYL GLYCERINE | Seppic (France) | 0,20000 |
| EMULIUM DELTA® | CETYL ALCOOL et GLYCERYL STEARATE et PEG-75 STEARATE et CETETH-20 and STEARETH-20 | Gattefossé (France) | 5,00000 |
| LANETTE® 16 | ALCOOL CETYLIQUE | Cognis -BASF | 1,00000 |
| | HUILE DE FIGUE DE BARBARIE | IeS Labo (France) | 0,16000 |
| MYRITOL ®318 | TRIGLYCERIDE CAPRYLIQUE/CAPRIQUE | Cognis -BASF (France) | 7,00000 |
| ARISTOFLEX ®AVC | COPOLYMERE ACRYLOYLDIMETHYL LAURATE/ AMMONIUM VP | Clariant (France) | 0,50000 |
| THALASSINE 2 | SOLUTION AQUEUSE DE THALASSINE-2 | BIOTECH MARINE (France) | 1,60000 |
| | PHENOXYETHANOL | | 0,30000 |
| | CHLORPHENESINE | | 0,20000 |
| | SOLUTION HYDRO GLYCOLIQUE DE LYS | IeS Labo (France) | 0,06000 |
| SPIRULINE HS | SOLUTION HYDRO GLYCOLIQUE SPIRULINE | GREENTECHr (France) | 0,03000 |
| | HYALURONATE DE SODIUM | | 0,16000 |
| | HYDROXYDE DE SODIUM à 10% | | Qsp pH5,67 |
| | | | 100,00000 |

On a suivi le mode opératoire comprenant les étapes suivantes :

a) Dans un mélangeur chauffer à 80°C:

- EAU PURIFIEE,

- EDTA (agent conservateur), et
- CHLORPHENESINE (agent conservateur).

b) Mélanger et introduire le mélange:

- GLYCERINE (agent hydratant),
- HYALURONATE DE SODIUM,

c) Disperser sous agitation durant 30 minutes dans un fondoir dans lequel on a introduit et chauffé à 75°C :

- EMULIUM DELTA® (agent émulsifiant),
- LANETTE ®16 (agent émollient), et
- MYRITOL® 318 (agent émollient).

d) Introduire le contenu du fondoir dans le mélangeur et a jouter:

- HUILE DE FIGUE DE BARBARIE, et
- ARISTOFLEX® AVC (agent épaississant)

e) Homogénéiser 10 minutes et refroidir à 50°C puis incorporer:

- PHENOXYETHANOL (agent conservateur), et
- SENSIVA® SC 50(agent conservateur).

f) Homogénéiser et introduire:

- SOLUTION HYDRO GLYCOLIQUE DE LYS
- SOLUTION HYDRO ALCOOLIQUE DE SPIRULINE
- SOLUTION DE THALASSINE 2

g) Homogénéiser 10 minutes à 25°C et ajuster le Ph à 5,67 à l'aide d'une solution de soude (Na OH) à 10%.

2) GEL AQUEUX

On a préparé un gel à partir du mélange d'actifs CAAL-2 de composition suivante :

| NOM COMMERCIAL | NOM INCI | FOURNISSEUR | % en poids |
|---|---|---|---|
| EAU PURIFIEE | | | 91,79000 |
| ULTREZ ®10 | CARBOMERE | Lubrizol (Fance) | 0,20000 |
| GLYCERINE | GLYCERINE | | 5,00000 |
| GEOGARD ®ULTRA | GLUCONOLACTONE, CALCIUM GLUCONATE, SODIUM BENZOATE | Lonza (CH°) | 0,50000 |
| JAGUAR® 105 HP TRIETHANOLAMINE | HYDROXYPROPYL GUAR TRIETHANOLAMINE | Rhodia (France) Interchimie (France) | 0,30000 0,20000 |
| HUILE DE FIGUE DE BARBARIE | | les labo (France) | 0,16000 |
| SOLUTION DE THALASSINE 2 | | Biotech marine (France) | 1,60000 |
| SOLUTION HYDROGLYCOLIQUE DE FLEUR DE LYS | | les labo (France) | 0,06000 |

(suite)

| NOM COMMERCIAL | NOM INCI | FOURNISSEUR | % en poids |
|---|---|---|---|
| SOLUTION HYDROGLYCOLIQUE SPIRULINE HS | | Greentech (France) | 0,03000 |
| HYALURONATE DE SODIUM | | Biophil (France) ® | 0,16000 |
| | | 100,000 | |

La composition de gel ci-dessus a été préparée en suivant le mode opératoire comprenant les étapes suivantes.

a) préparation d'une phase A

Saupoudrer l'ULTREZ ®10 (agent gélifiant) dans une partie d'eau et laisser reposer 20 minutes.

b) préparation d'une phase B

- Dans un mélangeur chauffer la glycérine (agent hydratant) avec une partie d'eau à 60°C et dissoudre GEOGARD® ULTRA (agent conservateur);
- Ajouter le HYALURONATE DE SODIUM et disperser sous agitation pendant 30 minutes, et
- A 40°C ajouter JAGUAR® 105 HP (agent épaississant) et homogénéiser.

c) Mélange des deux phases A et B.

- Ajouter la phase A à la phase B sous agitation et laisser reposer pendant 40 minutes,
- Neutraliser (Ph=7) avec le TRIETHANOLAMINE (ajusteur de Ph), et
- Introduire: les 4 ingrédients actifs SOLUTION DE FLEUR DE LYS, SOLUTION DE SPIRULINE, SOLUTION DE THALASSINE 2 et HUILE DE FIGUE DE BARBARIE ;
- Homogénéiser 10 minutes.

## Revendications

1.  Complexe actif anti âge pour la peau comprenant au moins les éléments suivants:

    - thalassine, extraite de *Tripleurospermum Maritimum*,
    - huile de pépins de figue de barbarie, et
    - acide hyaluronique ou sel de celui-ci.

2.  Complexe selon la revendication 1 sous forme liquide comprenant :

    - 0,2 à 3% en poids d'une solution aqueuse de thalassine à 2% en poids de thalassine,
    - 0,02 à 2% en poids d'huile de figue de barbarie, et
    - 0,02 à 0,2% en poids d'acide hyaluronique ou sel de celui-ci.

3.  Complexe selon l'une des revendications 1 ou 2 comprenant en outre au moins les éléments suivants :

    - extrait de fleur de lys blanc *Lilium candidum,* de préférence une solution hydro glycolique de fleur de Lys blanc *Lilium candidum,* et.
    - spiruline, extraite de *Spirula Maxima,* de préférence une solution hydro glycolique de spiruline.

**4.** Complexe selon la revendication 3 sous forme liquide comprenant :

 - 0,02 à 0,15% en poids de solution hydro glycolique contenant 5% en poids de dit extrait de Lys blanc.
 - 0,002 à 0,05% en poids de solution hydro glycolique contenant 1% en poids de spiruline.

**5.** Complexe selon l'une des revendications 1 à 4 sous forme liquide comprenant au moins :

 - 1 à 2% en poids de solution aqueuse de thalassine à 2% en poids de thalassine,
 - 0,1 à 0,2% en poids de dite huile de figue de barbarie, et
 - 0,1 à 0,2% en poids d'acide hyaluronique ou sel de celui-ci.

**6.** Complexe selon l'une des revendications 1 à 5 sous liquide de solution comprenant en outre au moins :

 - 0,01 à 0,1% en poids solution hydro glycolique contenant 5% en poids de dit extrait de Lys blanc, et
 - 0,01 à 0,1% en poids de solution hydro glycolique contenant 1% en poids de spiruline.

**7.** Complexe selon l'une des revendications 1 à 6 sous forme liquide comprenant :

 - 1,6 % en poids de dite solution de thalassine
 - 0,16 % en poids de dite huile de figue de barbarie
 - 0,16 mg/ml d'acide hyaluronique.

**8.** Complexe selon la revendication 7 sous forme liquide comprenant :

 - 1,6 % en poids de dite solution de thalassine,
 - 0,16 % en poids d'huile de figue de barbarie,
 - 0,16 % en poids d'acide hyaluronique,
 - 0,06 % en poids de dite solution hydro glycolique de dit extrait de lys blanc, et
 - 0,03 % en poids de dite solution hydro glycolique de spiruline.

**9.** Produit cosmétique ou dermatologique comprenant un dit complexe selon l'une des revendications 1 à 8 en combinaison avec des supports et/ou excipients cosmétiquement ou pharmaceutiquement acceptables.

**10.** Produit cosmétique antivieillissement selon la revendication 9 **caractérisé en ce qu'**il est formulé pour une application topique sous forme de gel, lotion, crème, pommade, savon, pâte pour masque.


**Patentansprüche**

**1.** Anti-Aging Wirkstoffkomplex für die Haut, der zumindest die folgenden Bestandteile umfasst:

 - Thalassin, das aus *Tripleurospermum maritimum* extrahiert ist,
 - Kaktusfeigenkernöl und
 - Hyaluronsäure oder Salz davon.

**2.** Komplex gemäß Anspruch 1 in flüssiger Form, umfassend:

 - 0,2 bis 3 Gew.% einer wässrigen Thalassinlösung mit 2 Gew.% Thalassin,
 - 0,02 bis 2 Gew.% Kaktusfeigenöl und
 - 0,02 bis 0,2 Gew.% Hyaluronsäure oder Salz davon.

**3.** Komplex gemäß einem der Ansprüche 1 oder 2, der ferner zumindest die folgenden Bestandteile umfasst:

 - Blütenextrakt aus weißer Lilie *Lilium candidum,* vorzugsweise eine wässrig-glycolische Lösung aus Blüten aus weißer Lilie *Lilium candidum,* und

 - Spirulina, die aus der *Spirulina maxima* extrahiert ist, vorzugsweise eine wässrig-glycolische Spirulina-Lösung.

**4.** Komplex gemäß Anspruch 3 in flüssiger Form, umfassend:

- 0,02 bis 0,15 Gew.% wässrig-glycolische Lösung, die 5 Gew.% Extrakt aus weißer Lilie enthält,
- 0,002 bis 0,15 Gew.% wässrig-glycolische Lösung, die 1 Gew.% Spirulina enthält.

**5.** Komplex gemäß einem der Ansprüche 1 bis 4 in flüssiger Form, der zumindest umfasst:

- 1 bis 2 Gew.% wässrige Thalassinlösung mit 2 Gew.% Thalassin,
- 0,1 bis 0,2 Gew.% Kaktusfeigenöl und
- 0,1 bis 0,2 Gew.% Hyaluronsäure oder Salz davon.

**6.** Komplex gemäß einem der Ansprüche 1 bis 5 in flüssiger Form, der ferner zumindest umfasst:

- 0,01 bis 0,1 Gew.% wässrig-glycolische Lösung, die 5 Gew.% Extrakt aus weißer Lilie enthält,
- 0,01 bis 0,1 Gew.% wässrig-glycolische Lösung, die 1 Gew.% Spirulina enthält.

**7.** Komplex gemäß einem der Ansprüche 1 bis 6 in flüssiger Form, umfassend:

- 1,6 Gew.% Thalassinlösung
- 0,16 Gew.% Kaktusfeigenöl
- 0,16 mg/ml Hyaluronsäure.

**8.** Komplex gemäß Anspruch 7 in flüssiger Form, umfassend:

- 1,6 Gew.% Thalassinlösung,
- 0,16 Gew.% Kaktusfeigenöl,
- 0,16 Gew.% Hyaluronsäure,
- 0,06 Gew.% wässrig-glycolische Lösung des Extrakts aus weißer Lilie und
- 0,03 Gew.% wässrig-glycolische Spirulina-Lösung.

**9.** Kosmetisches oder dermatologisches Produkt, umfassend einen Komplex gemäß einem der Ansprüche 1 bis 8 in Kombination mit Trägern und/oder Hilfsstoffen, die kosmetisch oder pharmazeutisch akzeptabel sind.

**10.** Kosmetisches Anti-Aging-Produkt gemäß Anspruch 9, **dadurch gekennzeichnet, dass** es für eine topische Anwendung in Form von Gel, Lotion, Creme, Salbe, Seife, Paste für eine Maske formuliert ist.

**Claims**

**1.** An anti-age active complex for the skin comprising at least the following elements:

- thalassin, extracted from *Tripleurospermum Maritimum*,
- prickly pear seed oil, and
- hyaluronic acid or salt thereof.

**2.** The complex according to claim 1 in liquid form comprising:

- 0.2 to 3% by weight of an aqueous solution of thalassin with 2% by weight of thalassin,
- 0.02 to 2% by weight of prickly pear seed oil, and
- 0.02 to 0.2% by weight of hyaluronic acid or salt thereof.

**3.** The complex according to one of claims 1 or 2, further comprising at least the following elements:

- extract from white lily flower *Lilium candidum,* preferably a hydroglycolic solution of white lily flower *Lilium candidum,* and,
- spirulin, extracted from *Spirula Maxima,* preferably a hydroglycolic solution of spirulin.

**4.** The complex according to claim 3 in liquid form comprising:

- 0.02 to 0.15% by weight of hydroglycolic solution containing 5% by weight of said white lily flower extract,
- 0.002 to 0.05% by weight of hydroglycolic solution containing 1% by weight of spirulin.

5. The complex according to one of claims 1 to 4 in liquid form comprising at least:

- 1 to 2% by weight of an aqueous solution of thalassin with 2% by weight of thalassin,
- 0.1 to 0,2% by weight of said prickly pear oil, and
- 0.1 to 0.2% by weight of hyaluronic acid or salt thereof.

6. The complex according to one of claims 1 to 5 in a liquid solution further comprising at least:

- 0.01 to 0.1% by weight of a hydroglycolic solution containing 5% by weight of said white lily extract, and
- 0.01 to 0.1% by weight of a hydroglycolic solution containing 1% by weight of spirulin.

7. The complex according to one of claims 1 to 6 in liquid form comprising:

- 1.6% by weight of said thalassin solution,
- 0.16% by weight of said prickly pear oil,
- 0.16 mg/ml of hyaluronic acid.

8. The complex according to claim 7 in liquid form comprising:

- 1.6% by weight of said thalassin solution,
- 0.16% by weight of prickly pear oil,
- 0.16% by weight of hyaluronic acid,
- 0.06% by weight of said hydroglycolic solution of said white lily extract, and
- 0.03% by weight of said hydroglycolic solution of spirulin.

9. A cosmetic or dermatological product comprising one said complex according to one of claims 1 to 8 in combination with cosmetically or pharmaceutically acceptable supports and/or excipients.

10. The anti-ageing cosmetic product according to claim 9, **characterized in that** it is formulated for a topical application as a gel, lotion, cream, pomade, soap, paste for a mask.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 02060394 A
- WO 2009050866 A
- DE 102009045981
- US 2009116211 A

**Littérature non-brevet citée dans la description**

- Normal Keratinization in a Spontaneously Immortalized Aneuploid Human Keratinocyte Cell Line N.E Fusenig. *J. Cell. Biology,* 1988, 106
- **A. GOMES et al.** Fluorescence probes used for détection of reactive oxygen species. *J. Biochem. Biophys. Methods,* 2005, vol. 65, 45-80